# EUROPÄISCHE PATENTSCHRIFT

(11) **EP 2 094 232 B1**
(45) Veröffentlichungstag und Bekanntmachung des Hinweises auf die Patenterteilung: **07.04.2010**
(21) Anmeldenummer: 07819760.5
(22) Anmeldetag: 12.11.2007
(51) Int. Cl.: A61K 8/19, A61K 8/97, A61Q 19/00

(54) **KÖRPER- ODER SCHÖNHEITSPFLEGEMITTEL**
BODY CARE OR BEAUTY CARE PRODUCT
AGENT POUR SOINS CORPORELS OU POUR SOINS DE BEAUTÉ

(30) Priorität: 17.11.2006 DE 102006054653
(43) Veröffentlichungstag der Anmeldung: 02.09.2009
(73) Patentinhaber: ADA Cosmetic GmbH, 77694 Kehl/Baden (DE)
(72) Erfinder: SIETZ, Manfred, 37627 Wangelnstedt (DE)
(74) Vertreter: Maucher, Wolfgang
(86) Internationale Anmeldenummer: PCT/EP2007/009771
(87) Internationale Veröffentlichungsnummer: WO 2008/058693

(56) Entgegenhaltungen:
- EP-A- 0 000 968
- WO-A-00/50045
- JP-A- 2004 073 964
- US-A- 5 932 251
- DATABASE WPI Week 200476 Derwent Publications Ltd., London, GB; AN 2004-768758 XP002474946 & JP 2004 285166 A (TAGAWA M) 14. Oktober 2004 (2004-10-14)
- DATABASE WPI Week 200675 Derwent Publications Ltd., London, GB; AN 2006-726925 XP002474947 & WO 2006/101106 A (SHETECH CO LTD) 28. September 2006 (2006-09-28)
- DATABASE WPI Week 200724 Derwent Publications Ltd., London, GB; AN 2007-237323 XP002474948 & KR 2006 108 789 A (FINECO LTD) 18. Oktober 2006 (2006-10-18)
- DATABASE WPI Week 200273 Derwent Publications Ltd., London, GB; AN 2002-679740 XP002474949 & KR 2002 035 231 A (AN J O) 11. Mai 2002 (2002-05-11)
- DATABASE WPI Week 200273 Derwent Publications Ltd., London, GB; AN 2002-679741 XP002474950 & KR 2002 035 232 A (AN J O) 11. Mai 2002 (2002-05-11)
- DATABASE WPI Week 200113 Derwent Publications Ltd., London, GB; AN 2001-123342 XP002474951 & ZA 200 000 192 A (JULLIENNE J R J) 27. September 2000 (2000-09-27)

## Beschreibung

Die Erfindung betrifft ein Mittel zur Körper- oder Schönheitspflege, das einen Anteil eines kolloidalen Edelmetalls enthält.

Man kennt bereits verschiedene Körper- oder Schönheitspflegemittel, in denen ein Anteil an Silbersalzen oder kolloidalem Silber enthalten ist. Überwiegend haben diese Silbersalze, die in geringen Mengen zum Einsatz kommen, den Zweck, in dem betreffenden Körper- oder Schönheitspflegemittel als Konservierungsmittel zu dienen. Da lediglich Silberchlorid in der Kosmetik-Verordnung zur Konservierung zugelassen ist, ist der Einsatz anderer Silbersalze beziehungsweise kolloidalen Silbers - nicht zuletzt auch wegen der physiologisch bedenklichen Wirkung von Silber - zur Verwendung in kosmetischen Mitteln zum Zwecke der Konservierung verboten. Da der Einsatz von Silber zu anderen Zwecken erlaubt ist, werden den über Silberchlorid hinaus verwendeten Silbersalzen eine medizinische Wirkung unterlegt.
Sofern der Einsatz solcher Silbersalze medizinischen Zwecken dienen soll, kommt ein solcher Einsatz jedoch nur als Akutmittel in Betracht.

Es besteht daher die Aufgabe, ein Körper- oder Schönheitspflegemittel zu schaffen, das auf den Körper des Anwenders eine gesundheitsfördernde und körperpflegende Wirkung hat, welche gegebenenfalls auch die Wirkung anderer Wirksubstanzen vervielfacht.

Die erfindungsgemäße Lösung dieser Aufgabe besteht bei dem Körper- oder Schönheitspflegemittel der eingangs erwähnten Art insbesondere darin, dass das Körper- oder Schönheitspflegemittel einen Anteil an kolloidalem Gold in Verbindung mit zumindest einem pflanzlichen und/oder ätherischen Öl oder Extrakt und/oder einem Biopolymer enthält.

Das erfindungsgemäße Körper- oder Schönheitspflegemittel enthält einen Anteil an kolloidalem Gold, der aufgrund seiner gesundheitsfördernden und körperpflegenden Wirkung als Wirkstoff dient. Um die-im kolloidalem Gold enthaltenen kleinsten Goldpartikel in ihrer kolloid-dispersen Phase lagern zu können, ist das kolloidale Gold in Verbindung mit zumindest einem pflanzlichen und/oder ätherischen Öl oder Extrakt und/oder einem Biopolymer enthalten. Es hat sich überraschend gezeigt, dass ein solcher Inhaltsstoff eine stark stabilisierende Wirkung auf das kolloidale Gold hat, die nun auch einen kommerziellen Einsatz ermöglichen. Die im erfindungsgemäßen Körper- oder Schönheitspflegemittel enthaltenen Goldpartikel haben eine immunsteigernde und entzündungshemmende Wirkung, regen die Zellbildung in der Lederhaut an, können aufgrund ihres starken negativen Oberflächenpotentials und ihrer großen aktiven Oberfläche überschüssige freie Radikale abfangen, steigern die Wirkung gegebenenfalls weiterer Inhalts- und Wirkstoffe, zeichnen sich durch eine spezifische Keimbindung und spezifische mikrobiozide Wirkung insbesondere gegen hauttypische Aknebakterien aus und wirken antiirritierend. Ferner haben die Goldpartikel eine ausgeprägte Trägereigenschaft.
Dabei sieht eine bevorzugte Ausführungsform gemäß der Erfindung vor, dass dem Körper- oder Schönheitspflegemittel Kamillenöl als pflanzliches ätherisches Öl oder Kamillenextrakt beigefügt ist. Das im erfindungsgemäßen Körper- oder Schönheitspflegemittel enthaltene Kamillenöl oder Kamillenextrakt zeichnet sich durch eine stark stabilisierende Wirkung auf das kolloidale Gold aus und begünstigt dessen gesundheitsfördernde und körperpflegende Wirkung in besonders vorteilhafter Weise.

Der Anteil an kolloidalem Gold im erfindungsgemäßen Körper- oder Schönheitspflegemittel kann vergleichsweise gering sein. Es hat sich gezeigt, dass das Körper- oder Schönheitspflegemittel beispielsweise auch nur 25 bis 100 ppm kolloidales Gold enthalten kann, und dennoch seine gesundheitsfördernde und körperpflegende Wirkung aufweist.

Das erfindungsgemäße Körper- oder Schönheitspflegemittel weist kleinste Goldpartikel in einer kolloid-dispersen Phase auf. Dabei ist es zweckmäßig, wenn die im Körper- oder Schönheitspflegemittel enthaltenen Goldpartikel eine durchschnittliche Größe von 5 bis 10 nm aufweisen.

Das erfindungsgemäße Körper- oder Schönheitspflegemittel kann zur äußeren und/oder inneren Anwendung bestimmt sein. Dabei sieht ein vorteilhafter Anwendungsbereich vor, dass das Körper- oder Schönheitspflegemittel als Nahrungsergänzungsmittel vorgesehen ist.

Ist das erfindungsgemäße Körper- oder Schönheitspflegemittel zur inneren Anwendung bestimmt, kann es vorteilhaft sein, wenn das Körper- oder Schönheitspflegemittel in Kapsel-, Dragee- oder Tropfenform einnehmbar ist.

Eine bevorzugte Ausführungsform gemäß der Erfindung sieht jedoch vor, dass das erfindungsgemäße Körper- oder Schönheitspflegemittel als Serum oder insbesondere als Emulsion ausgebildet ist.

Um die gesundheitsfördernde und körperpflegende Wirkung des erfindungsgemäßen Körper- oder Schönheitspflegemittels zu steigern, kann es vorteilhaft sein, wenn das Körper- oder Schönheitspflegemittel einen Anteil von zum Beispiel Koenzym Q10, Hyaloronsäure, Kollagen, Parakresse, Aloe Vera, Jojoba und/oder zumindest einem Vitamin enthält.

Eine bevorzugte Weiterbildung gemäß der Erfindung sieht vor, dass das Körper- oder Schönheitspflegemittel zumindest einen Anti-Aging-Wirkstoff enthält. Ist das erfindungsgemäße Körper- oder Schönheitspflegemittel als kosmetisches Produkt ausgebildet, so kann es vorteilhaft sein, wenn das Körper- oder Schönheitspflegemittel als Anti-Aging-Wirkstoff zumindest eines der nachfolgend genannten Bestandteile enthält:
Aminosäuren und Derivate, Proteine und hydrolysierte Form, Kollagene und Derivate, Elastin und Derivate, Keratin und Derivate, Peptide, Vitamine und Derivate, Mineralien und Derivate, Spurenelemente, Hydroxysäuren und Derivate, Pflanzenextrakte und deren isolierte Wirkstoffe, Hyaluronsäure und Derivate, Pflanzenöle, Ceramide, Sphingolipide, Cerebroside, Lichtschutzfilter, Enzyme und Derivate, Coenzyme und Derivate, Wirkstoffe aus Plankton, Wirkstoffe aus Hefen, Wirkstoffe aus Algen, Wirkstoffe aus Pilzen, Wirkstoffe aus Tang, Wirkstoffe aus sonstigen Meerestieren und -pflanzen, biotechnologisch hergestellte Rohstoffe, Pyrrolidoncarbonsäure und Derivate, Bestandteile des NMF (Natural Moisturizing Factor), Dextrin und Cyclodextrin und Derivate, Chitosan und Derivate, Chitin und Derivate, Zucker und Zuckeralkohole und Derivate, Lecithin und Derivate, Chondroitinsulfat, Glucosaminsulfat, Wirkstoffe, die durch Fermentation aus Mikroorganismen hergestellt werden, Wirkstoffe aus Mikroorganismen, Carnithin, Ursolsäure, Siliciumdioxid und Derivate, Stärke und Derivate, Silikone und Derivate, Nylon und Derivate, Polyethylen und Derivate, Polysaccharide und Derivate, Antioxidantien und Derivate, ATP und Derivate, Glykogen und Derivate sowie Kreatin und Derivate.

Ist das erfindungsgemäße Körper- oder Schönheitspflegemittel demgegenüber als Nahrungsergänzungsmittel vorgesehen, kann es zweckmäßig sein, wenn das Körper- oder Schönheitspflegemittel als Anti-Aging-Wirkstoff zumindest eines der nachfolgend genannten Bestandteile enthält:
Aminosäuren und Derivate, Proteine und hydrolysierte Form, Kollagene und Derivate, Elastin und Derivate, Keratin und Derivate, Peptide, Vitamine und Derivate, Mineralien und Derivate, Spurenelemente, Hydroxysäuren und Derivate, Pflanzenextrakte und deren isolierte Wirkstoffe, Hyaluronsäure und Derivate, Pflanzenöle, Ceramide, Sphingolipide, Cerebroside, Enzyme und Derivate, Coenzyme und Derivate, Wirkstoffe aus Plankton, Wirkstoffe aus Hefen, Wirkstoffe aus Algen, Wirkstoffe aus Pilzen, biotechnologisch hergestellte Rohstoffe, Pyrrolidoncarbonsäure und Derivate, Bestandteile des NMF (Natural Moisturizing Factor), Dextrin und Cyclodextrin und Derivate, Chitosan und Derivate, Zucker und Zuckeralkohole und Derivate, Lecithin und Derivate, Chondroitinsulfat, Glucosaminsulfat, Wirkstoffe, die durch Fermentation aus Mikroorganismen hergestellt werden, Wirkstoffe aus Mikroorganismen, Carnithin, Ursolsäure, Siliciumdioxid und Derivate, Stärke und Derivate, Polysaccharide und Derivate, Antioxidantien und Derivate, ATP und Derivate, Glykogen und Derivate, Kreatin und Derivate, Ballaststoffe und Probiotika.

Um das erfindungsgemäße Körper- oder Schönheitspflegemittel auch als Schleifmittel oder als Peeling verwenden zu können, kann es vorteilhaft sein, wenn das Körper- oder Schönheitspflegemittel zumindest ein Schleifmittel enthält.

Eine weitere vorteilhafte Ausführungsform gemäß der Erfindung sieht vor, dass das Körper- oder Schönheitspflegemittel mindestens einen absorbierenden Bestandteil, wie beispielsweise Maisstärke, aufweist. Ein solcher absorbierender Bestandteil kann auf der Hautoberfläche mattierend wirken und einen störenden Fettglanz verhindern. Darüber hinaus ist auch ein Zwei-Phasen-Körper- oder Schönheitspflegemittel möglich, das aufgrund seines Goldbestandteils eine antibakterielle Wirkung hat und mit Hilfe seiner absorbierenden Hilfsstoffe Fett aufnehmen kann.

Ein solches Körper- oder Schönheitspflegemittel verbessert das Hautgefühl und begünstigt eine Antiaging-Wirkung dieses Produktes.

Um die Hautverträglichkeit des erfindungsgemäßen Körper- oder Schönheitspflegemittels zu begünstigen ist es vorteilhaft, wenn das Körper- oder Schönheitspflegemittel wenigstens einen pHpuffernden Bestandteil hat.

Die Wirkung des im Körper- oder Schönheitspflegemittels enthaltenen Goldbestandteils kann noch begünstigt werden, wenn das Körper- oder Schönheitspflegemittel zumindest einen Korrosionsinhibitor enthält.

Um das erfindungsgemäße Körper- oder Schönheitspflegemittel auch gegen eine Aknehaut oder gegen Neurodermitis einsetzen zu können, ist es vorteilhaft, wenn im Körper- oder Schönheitspflegemittel wenigstens ein antimikrobieller Wirkstoff enthalten ist. Ein solcher antimikrobieller Wirkstoff kann auch vorteilhaft sein, um im Körper- oder Schönheitspflegemittel einen konservierenden Bestandteil vorzusehen.

Die Anti-Aging-Wirkung des erfindungsgemäßen Körper- oder Schönheitspflegemittels wird noch begünstigt, wenn das Körper- oder Schönheitspflegemittel mindestens ein Antioxidants aufweist.

Das erfindungsgemäße Körper- oder Schönheitspflegemittel zeichnet sich auch durch eine deodorierende Wirkung aus, wenn das Körper- oder Schönheitspflegemittel zumindest ein Deodorant hat.

Um das Körper- oder Schönheitspflegemittel auch als Konditioner und/oder als Haarpflegemittel verwenden zu können, ist es vorteilhaft, wenn, das Körper- oder Schönheitspflegemittel wenigstens ein Antistatika und/oder mindestens ein Konditioner enthält.

Bei Verwendung des erfindungsgemäßen Körper- oder Schönheitspflegemittels als Nahrungsergänzungsmittel kann es vorteilhaft sein, wenn im Körper- oder Schönheitspflegemittel zumindest ein Bindemittel enthalten ist.

Um eine Hautaufhellung mit Hilfe des Körper- oder Schönheitspflegemittels zu erreichen, ist es zweckmäßig, wenn das Körper- oder Schönheitspflegemittel wenigstens einen Hautaufheller hat.

Aufgrund der im erfindungsgemäßen Körper- oder Schönheitspflegemittel enthaltenen Goldbestandteile kann das erfindungsgemäße Körper- oder Schönheitspflegemittel eine lila Grundfarbe haben. Um einem solchen, gegebenenfalls auch unerwünschten lila Grundton entgegenzuwirken, kann es zweckmäßig sein, wenn das Körper- oder Schönheitspflegemittel zumindest einen produktfärbenden Bestandteil aufweist.

Mit Hilfe des erfindungsgemäßen Körper- oder Schönheitspflegemittels können auch die Haare eingefärbt werden, wenn das Körper- oder Schönheitspflegemittel wenigstens ein Färbemittel enthält.

Sofern im erfindungsgemäßen Körper- oder Schönheitspflegemittel beispielsweise ethanolische Lösungen enthalten sind, kann es vorteilhaft sein, wenn im Körper- oder Schönheitspflegemittel zumindest ein Vergällungsmittel vorgesehen ist.

Eine weitere vorteilhafte Ausführungsform gemäß der Erfindung sieht vor, dass im Körper- oder Schönheitspflegemittel wenigstens ein Enthaarungsmittel vorgesehen ist. Dabei wird einer hautreizenden Wirkung des Enthaarungsmittels durch den im Körper- oder Schönheitspflegemittel zusätzlich enthaltenen Goldbestandteil entgegengewirkt.

Um bei der Anwendung des erfindungsgemäßen Körper- oder Schönheitspflegemittels das Hautgefühl positiv zu beeinflussen, kann es vorteilhaft sein, wenn im Körper- oder Schönheitspflegemittel wenigstens ein Emollient oder dergleichen weichmachender Wirkstoff enthalten ist.

Sofern das erfindungsgemäße Körper- oder Schönheitspflegemittel in einer cremigen Darreichungsform angeboten werden soll, kann es vorteilhaft sein, wenn das Körper- oder Schönheitspflegemittel zumindest einen Emulgator enthält.

Für eine im erfindungsgemäßen Körper- oder Schönheitspflegemittel gegebenenfalls enthaltene Basisemulsion kann es zweckmäßig sein, wenn das Körper- und Schönheitspflegemittel zumindest einen Emulsionsstabilisator aufweist.

Um den Anti-Aging-Effekt des erfindungsgemäßen Körper- oder Schönheitspflegemittels zu begünstigen, um eine Hautstraffung mit Hilfe dieses Körper- oder Schönheitspflegemittels zu erreichen, und um dem Körper- oder Schönheitspflegemittel erforderlichenfalls auch eine feuchtigkeitsspendende Wirkung zu geben, kann es zweckmäßig sein, wenn das Körper- oder Schönheitspflegemittel mindestens einen Filmbildner aufweist.

Vergleichbare Vorteile lassen sich erreichen, wenn im Körper- oder Schönheitspflegemittel wenigstens ein Feuchthaltemittel enthalten und insbesondere Glycerin als Basiswirkstoff vorgesehen ist.

Um den aufgrund des Goldbestandteils bewirkten Farbton des erfindungsgemäßen Körper- oder Schönheitspflegemittels abzumildern und um das Körper- oder Schönheitspflegemittel beispielsweise auch als Cremebad anbieten zu können, ist es vorteilhaft, wenn das Körper- oder Schönheitspflegemittel mindestens ein Trübungsmittel aufweist.

Um die vorteilhaften Wirkungen des erfindungsgemäßen Körper- oder Schönheitspflegemittels auch im Zahnpflegebereich und insbesondere bei einer Zahncreme anwenden zu können, ist es vorteilhaft, wenn im Körper- oder Schönheitspflegemittel zumindest ein Mundpflegemittel enthalten ist.

Die Wasch- und Reinigungswirkung des Körper- oder Schönheitspflegemittels wird begünstigt, wenn das Körper- oder Schönheitspflegemittel wenigstens ein Oxidationsmittel aufweist.

Die Lagerungsfähigkeit des erfindungsgemäßen Körper- oder Schönheitspflegemittels wird erhöht, wenn darin mindestens ein Konservierungsmittel vorgesehen ist.

Um mit Hilfe des erfindungsgemäßen Körper- oder Schönheitspflegemittels auch körper- und schönheitspflegende Schäume herstellen zu können, sieht eine bevorzugte Ausführungsform gemäß der Erfindung vor, dass im Körper- oder Schönheitspflegemittel mindestens ein schaumbildendes Treibgas vorgesehen ist.

Sofern mit Hilfe des erfindungsgemäßen Körper- oder Schönheitspflegemittels auch eine optimierte Hautreizung angestrebt wird, kann es vorteilhaft sein, wenn das Körper- oder Schönheitspflegemittel wenigstens ein Lösungsmittel enthält.

Die hohe Reinigungswirkung des Körper- oder Schönheitspflegemittels wird begünstigt, wenn im Körper- oder Schönheitspflegemittel zumindest ein Tensid vorgesehen ist.

Die Radikalfängereigenschaften lassen sich verbessern und der durch die Goldbestandteile bereits bewirkte UV-Schutz wird noch zusätzlich verstärkt, wenn im Körper- oder Schönheitspflegemittel zumindest ein Bestandteil als Lichtschutzfilter vorgesehen ist. Ein solches Körper- oder Schönheitspflegemittel, bei welchem ein Bestandteil als Lichtschutzfilter dient, zeichnet sich durch einen wesentlich verbesserten Zellschutz aus. Sofern das erfindungsgemäße Körper- oder Schönheitspflegemittel beispielsweise auch als Gel angeboten werden soll, kann die Viskosität des Körper- oder Schönheitspflegemittels in üblicher Weise eingestellt werden, wenn das Körper- oder Schönheitspflegemittel mindestens ein Verdickungsmittel enthält.

Sofern das erfindungsgemäße Körper- oder Schönheitspflegemittel auch als Nahrungsergänzungsmittel vertrieben und dazu beispielsweise als Bonbon angeboten werden soll, kann es vorteilhaft sein, wenn im Körper- oder Schönheitspflegemittel auch Honig enthalten ist.

Nachstehend wird die Erfindung anhand dreier Rezepturen noch näher beschrieben, die sich als besonders vorteilhaft erwiesen haben:
1. Die Rezeptur für eine erfindungsgemäß ausgebildete Augencreme sieht beispielsweise die nachfolgend aufgelisteten Bestandteile vor: AQUA, GLYCERIN, OLEYL ERUCATE, OCTYLDODECANOL, CETEARYL ALCOHOL, GLYCINE SOJA. OIL, DISTARCH PHOSPHATE, CAPRYLIC/CAPRIC TRIGLYCERIDE, CETEARYL GLUCOSIDE, HYDROXYETHYL ACRYLATE/SODIUM ACRYLOYL DIMETHYL TAURATE COPOLYMER, DECYL OLEATE, BIOSACCARIDE GUM-1, CHAMOMILLA RECUTITA EXTRACT, GLYCINE SOJA PROTEIN, GOLD, HYDROGENATED COTTONSEED OIL, HYDROLYZED RICE PROTEIN, OXIDO REDUCTASES, POTASSIUM CETYL PHOSPHATE, POTASSIUM HYDROXIDE, PARFUM, BUTYLPHENYL METHYLPROPIONAL, HEXYL CINNAMAL, LIMONENE, LINALOOL, PHENOXYETHANOL, ETHYLPARABEN, METHYLPARABEN, PROPYLPARABEN.
2. Ein Serum, das eine hautentspannende Wirkung hat und unerwünschten Gesichtsfalten entgegenwirken soll, sieht die nachfolgenden Bestandteile vor: AQUA, GLYCERIN, PROPYLHEPTYL CAPRYLATE, CAPRYLIC/CAPRIC TRIGLYCERIDE, DISTARCH PHOSPHATE, ACRYLATES/C10-30 ALKYL ACRYLATE CROSSPOLYMER, ALCOHOL, BIOSACCHARIDE GUM-1, CARNOSINE, CETEARYL ALCOHOL, CETEARYL GLUCOSIDE, CHAMOMILLA RECUTITA EXTRACT, GOLD, HYDROGENATED PALM GLYCERIDES, HYDROGENATED PALM KERNEL GLYCERIDES, LECITHIN, POTASSIUM CETYL PHOSPHATE, POTASSIUM HYDROXIDE, SILYBUM MARIANUM FRUIT EXTRACT, TOCOPHEROL, XANTHAN GUM, PARFUM, BUTYLPHENYL METHYLPROPIONAL, HEXYL CINNAMAL, LIMONENE, LINALOOL, PHENOXYETHANOL, ETHYLPARABEN, METHYLPARABEN, PROPYLPARABEN.
3. Ein Körper- oder Schönheitspflegemittel, das als Gesichtscreme vorgesehen ist, lässt sich beispielsweise anhand der nachfolgend beschriebenen Rezeptur herstellen: AQUA, GLYCERIN, DECYL OLEATE, CAPRYLIC/CAPRIC TRIGLYCERIDE, CETEARYL GLUCOSIDE, GLYCERYL STEARATE, CETEARYL ALCOHOL, TOCOPHERYL ACETATE, SHOREA STENOPTERA BUTTER, BUTYROSPERMUM PARKII BUTTER, ASCORBYL PALMITATE, CARNOSINE, CETYL ALCOHOL, CHAMOMILLA RECUTITA EXTRACT, GLYCINE SOJA STEROL, GOLD, OLUS OIL, POTASSIUM CETYL PHOSPHATE, RETINYL PALMITATE, SODIUM CARBOXYMETHYL BETAGLUCAN, SODIUM LACTATE, SODIUM STEAROYL LACTYLATE, UBIQUINONE, XANTHAN GUM, PARFUM, BUTYLPHENYL METHYLPROPIONAL, HEXYL CINNAMAL, LIMONENE, LINALOOL, PHENOXYETHANOL, ETHYLPARABEN, METHYLPARABEN, PROPYLYPARABEN.

## Patentansprüche

1. Mittel zur Körpers oder Schönheitspflege, das einen Anteil eines kolloidalen Edelmetalls enthält, **dadurch gekennzeichnet**, das das Körper- oder Schönheitspflegemittel einen Anteil an kolloidalem Gold in Verbindung mit Kamillenöl oder Kamillenextrakt enthält.

2. Mittel zur Körper- oder Schönheitspflege nach Anspruch 1 zur Behandlung von Neurodermitis und / oder Hautirritationen.

3. Körper- oder Schönheitspflegemittel nach Anspruch 1 oder 2, **dadurch gekennzeichnet, dass** das Körper- oder Schönheitspflegemittel 25 bis 100 ppm kolloidales Gold enthält.

4. Körper- oder Schönheitspflegemittel nach einem der Ansprüche 1 bis 3, **dadurch gekennzeichnet, dass** die im Körper- oder Schönheitspflegemittel enthaltenen Goldpartikel eine durchschnittliche Größe von 5 bis 10 nm aufweisen.

5. Körper- oder Schönheitspflegemittel nach einem der Ansprüche 1 bis 4, **dadurch gekennzeichnet, dass** das Körper- oder Schönheitspflegemittel zur äußeren und/oder inneren Anwendung bestimmt ist.

6. Körper- oder Schönheitspflegemittel nach einem der Ansprüche 1 bis 5, **dadurch gekennzeichnet, dass** das Körper- oder Schönheitspflegemittel als Nahrungsergänzungsmittel vorgesehen ist.

7. Körper- oder Schönheitspflegemittel nach einem der Ansprüche 1 bis 6, **dadurch gekennzeichnet, dass** das Körper- oder Schönheitspflegemittel in Kapsel-, Dragee- oder Tropfenform einnehmbar ist.

8. Körper- oder Schönheitspflegemittel nach einem der Ansprüche 1 bis 6, **dadurch gekennzeichnet, dass** das Körper- oder Schönheitspflegemittel als Serum oder insbesondere als Emulsion ausgebildet ist.

9. Körper- oder Schönheitspflegemittel nach einem der Ansprüche 1 bis 8, **dadurch gekennzeichnet, dass** das Körper- oder Schönheitspflegemittel einen Anteil an Koenzym Q10, Hyaluronsäure, Kollagen, Parakresse, Aloe Vera, Jojoba und/oder zumindest einem Vitamin enthält.

10. Körper- oder Schönheitspflegemittel nach einem der Ansprüche 1 bis 9, **dadurch gekennzeichnet, dass** das Körper- oder Schönheitspflegemittel zumindest einen Anti-Aging-Wirkstoff enthält.

11. Körper- oder Schönheitspflegemittel nach einem der Ansprüche 1 bis 10, **dadurch gekennzeichnet, dass** das Körper- oder Schönheitspflegemittel zumindest einen Korrosionsinhibitor enthält.

12. Körper- oder Schönheitspflegemittel nach einem der Ansprüche 1 bis 11, **dadurch gekennzeichnet, dass** im Körper- oder Schönheitspflegemittel wenigstens ein antimikrobieller Wirkstoff enthalten ist.

13. Körper- oder Schönheitspflegemittel nach einem der Ansprüche 1 bis 12, **dadurch gekennzeichnet, dass** im Körper- oder Schönheitspflegemittel zumindest ein Bestandteil als Lichtschutzfilter vorgesehen ist.

14. Körper- oder Schönheitspflegemittel nach einem der Ansprüche 1 bis 13, **dadurch gekennzeichnet, dass** das Körper- oder Schönheitspflegemittel mindestens ein Antioxidant aufweist.

15. Körper- oder Schönheitspflegemittel nach einem der Ansprüche 1 bis 14, **dadurch gekennzeichnet, dass** im Körper- oder Schönheitspflegemittel wenigstens ein Feuchthaltemittel enthalten ist.

## Claims

1. Product for body care or beauty care which contains an amount of a colloidal noble metal, **characterised in that** the body care or beauty care product contains an amount of colloidal gold in conjunction with camomile oil or camomile extract.

2. Product for body care or beauty care according to claim 1 for treating neurodermatitis and/or skin irritations.

3. Body care or beauty care product according to claim 1 or claim 2, **characterised in that** the body care or beauty care product contains 25 to 100 ppm of colloidal gold.

4. Body care or beauty care product according to one of claims 1 to 3, **characterised in that** the gold particles contained in the body care or beauty care product have an average size of 5 to 10 nm.

5. Body care or beauty care product according to one of claims 1 to 4, **characterised in that** the body care or beauty care product is intended for external and/or internal use.

6. Body care or beauty care product according to one of claims 1 to 5, **characterised in that** the body care or beauty care product is provided as a food supplement.

7. Body care or beauty care product according to one of claims 1 to 6, **characterised in that** the body care or beauty care product can be taken in capsule, coated tablet or droplet form.

8. Body care or beauty care product according to one of claims 1 to 6, **characterised in that** the body care or beauty care product is in the form of a serum or more particularly an emulsion.

9. Body care or beauty care product according to one of claims 1 to 8, **characterised in that** the body care or beauty care product contains an amount of coenzyme Q10, hyaluronic acid, collagen, paracress, aloe vera, jojoba and/or at least one vitamin.

10. Body care or beauty care product according to one of claims 1 to 9, **characterised in that** the body care or beauty care product contains at least one anti-ageing active substance.

11. Body care or beauty care product according to one of claims 1 to 10, **characterised in that** the body care or beauty care product contains at least one corrosion inhibitor.

12. Body care or beauty care product according to one of claims 1 to 11, **characterised in that** at least one antimicrobial active substance is present in the body care or beauty care product.

13. Body care or beauty care product according to one of claims 1 to 12, **characterised in that** at least one ingredient acting as a UV filter is present in the body care or beauty care product.

14. Body care or beauty care product according to one of claims 1 to 13, **characterised in that** the body care or beauty care product contains at least one antioxidant.

15. Body care or beauty care product according to one of claims 1 to 14, **characterised in that** at least one moisturiser is present in the body care or beauty care product.

## Revendications

1. Produit de soin du corps ou de beauté, qui contient pour une part un métal noble colloïdal, **caractérisé en ce que** le produit de soin du corps ou de beauté contient pour une part de l'or colloïdal lié à de l'huile de camomille ou à de l'extrait de camomille.

2. Produit de soin du corps ou de beauté selon la revendication 1 destiné au traitement de l'eczéma atopique et/ou des irritations de la peau.

3. Produit de soin du corps ou de beauté selon la revendication 1 ou 2, **caractérisé en ce que** le produit de soin du corps ou de beauté contient de 25 à 100 ppm d'or colloïdal.

4. Produit de soin du corps ou de beauté selon l'une quelconque des revendications 1 à 3, **caractérisé en ce que** les particules d'or contenues dans le produit de soin du corps ou de beauté présentent une taille moyenne allant de 5 à 10 nm.

5. Produit de soin du corps ou de beauté selon l'une quelconque des revendications 1 à 4, **caractérisé en ce que** le produit de soin du corps ou de beauté est conçu pour une utilisation externe et/ou interne.

6. Produit de soin du corps ou de beauté selon l'une quelconque des revendications 1 à 5, **caractérisé en ce que** le produit de soin du corps ou de beauté est prévu pour être utilisé comme complément alimentaire.

7. Produit de soin du corps ou de beauté selon l'une quelconque des revendications 1 à 6, **caractérisé en ce que** le produit de soin du corps ou de beauté peut être ingéré sous forme de gélules, de pastilles ou de gouttes.

8. Produit de soin du corps ou de beauté selon l'une quelconque des revendications 1 à 6, **caractérisé en ce que** le produit de soin du corps ou de beauté est préparé sous forme de sérum ou en particulier sous forme d'émulsion.

9. Produit de soin du corps ou de beauté selon l'une quelconque des revendications 1 à 8, **caractérisé en ce que** le produit de soin du corps ou de beauté contient pour une part de la co-enzyme Q10, de l'acide hyaluronique, du collagène, du cresson de Para, de l'aloe vera, du jojoba et/ou au moins une vitamine.

10. Produit de soin du corps ou de beauté selon l'une quelconque des revendications 1 à 9, **caractérisé en ce que** le produit de soin du corps ou de beauté contient au moins une substance active anti-âge.

11. Produit de soin du corps ou de beauté selon l'une quelconque des revendications 1 à 10, **caractérisé en ce que** le produit de soin du corps ou de beauté contient au moins un inhibiteur de la corrosion.

12. Produit de soin du corps ou de beauté selon l'une quelconque des revendications 1 à 11, **caractérisé en ce que** le produit de soin du corps ou de beauté contient au moins une substance active anti-microbienne.

13. Produit de soin du corps ou de beauté selon l'une quelconque des revendications 1 à 12, **caractérisé en ce qu'**au moins un constituant du produit de soin du corps ou de beauté est prévu comme filtre de protection contre la lumière.

14. Produit de soin du corps ou de beauté selon l'une quelconque des revendications 1 à 13, **caractérisé en ce que** le produit de soin du corps ou de beauté présente au moins un anti-oxydant.

15. Produit de soin du corps ou de beauté selon l'une quelconque des revendications 1 à 14, **caractérisé en ce que** le produit de soin du corps ou de beauté contient au moins un agent hydratant.
